# EUROPÄISCHE PATENTANMELDUNG

(11) **EP 1 576 981 A2**
(43) Veröffentlichungstag der Anmeldung: **21.09.2005**
(21) Anmeldenummer: 05005747.0
(22) Anmeldetag: 16.03.2005
(51) Int. Cl.: A61M 25/10

(54) **Steuergerät für einen Ballonkatheder**

(30) Priorität: 16.03.2004 DE 102004012801
(71) Anmelder: WL-tec GmbH, 97877 Wertheim (DE)
(72) Erfinder: Lutz, Walter, 97877 Wertheim (DE); Weiss, Michael, 97907 Hasloch (DE)
(74) Vertreter: Zinnecker, Armin

(57) **Zusammenfassung**

Ein Steuergerät dient zum Betreiben eines Ballonkatheters. Um ein derartiges Steuergerät zu verbessern umfaßt es ein Gehäuse (6) und eine Kolben-ZylinderEinheit (16), die mit dem Gehäuse (6) lösbar verbindbar ist (Fig. 1).

## Beschreibung

Die Erfindung betrifft ein Steuergerät für einen Ballonkatheter und eine Kolben-Zylinder-Einheit für ein derartiges Steuergerät.

Ballonkatheter werden in verschiedenen medizinischen und anderen Bereichen verwendet. Sie umfassen eine Druckleitung, einen Gehäuseteil und einen Ballon, der durch innere Druckeinwirkung vergrößerbar bzw. aufblasbar ist. Als Druckmedium wird im allgemeinen eine Flüssigkeit verwendet.

Das erfindungsgemäße Steuergerät dient zum Betreiben eines derartigen Ballonkatheters. Es dient insbesondere dazu, den Ballonkatheter mit einem Druckmedium, insbesondere einer unter Druck stehenden Flüssigkeit, zu versorgen.

Vorbekannte Steuergeräte für Ballonkatheter sind als Einmalgeräte ausgestaltet. Sie können nur unter unverhältnismäßig großen Schwierigkeiten und/oder Kosten oder auch überhaupt nicht sterilisiert werden. Eine wiederholte Verwendung der vorbekannten Steuergeräte ist nicht möglich.

Aus der EP 565 045 A1 ist ein Steuer- und Aufblasgerät für einen Ballonkatheter bekannt, welches einen Zylinder und einen darin längsbeweglichen Kolben umfaßt.

Die DE-OS 41 15 683 offenbart eine Flüssigkeitsverdrängungs- und Druckerzeugungsvorrichtung für angioplastische Ballonkatheter mit einem Schnelllösemechanismus, der ein schnelles Verschieben einer mit einem Gewinde versehenen Schraubkolbenstange zur Erzielung einer Druckerhöhung und anschließend die wahlweise Kopplung des Mechanismus mit der Gewindefläche der Kolbenstange ermöglicht.

Aufgabe der Erfindung ist es, ein verbessertes Steuergerät für einen Ballonkatheter vorzuschlagen.

Erfindungsgemäß wird diese Aufgabe durch die Merkmale des Anspruchs 1 gelöst. Das Steuergerät für einen Ballonkatheter umfaßt ein Gehäuse und eine Kolben-Zylinder-Einheit, die mit dem Gehäuse lösbar verbindbar ist. Vor dem Gebrauch des Steuergeräts kann die Kolben-Zylinder-Einheit mit dem Gehäuse verbunden, insbesondere in das Gehäuse eingesetzt werden. Nach dem Gebrauch des Steuergerätes kann die Kolben-Zylinder-Einheit von dem Gehäuse gelöst, insbesondere aus dem Gehäuse herausgenommen werden. Das Gehäuse kann dann erneut verwendet werden. Es ist vorzugsweise derart ausgestaltet, daß es mit dem zum Betrieb des Ballonkatheters dienenden Druckmedium, insbesondere der Druckflüssigkeit, nicht in Verbindung kommt. Stattdessen oder zusätzlich kann das Gehäuse derart ausgestaltet sein, daß es sterilisierbar ist oder daß diejenigen Teile, die mit dem Druckmedium in Kontakt kommen, sterilisierbar sind. In dem Gehäuse kann eine Öffnung für die Kolben-Zylinder-Einheit vorgesehen sein. Die Kolben-Zylinder-Einheit kann vor dem Gebrauch durch diese Öffnung in das Gehäuse eingesetzt und mit dem Gehäuse verbunden werden. Nach dem Gebrauch kann die Kolben-Zylinder-Einheit von dem Gehäuse gelöst und durch die Öffnung aus dem Gehäuse herausgenommen werden.

Vorteilhafte Weiterbildungen sind in den Unteransprüchen beschrieben.

In dem Gehäuse ist vorzugsweise eine Druckmeßeinheit vorgesehen. In vielen Anwendungsfällen ist es wünschenswert oder erforderlich, den Druck in dem zum Betrieb des Ballonkatheters dienenden Druckmedium, insbesondere der Druckflüssigkeit, zu messen, wobei eine laufende bzw. ununterbrochene Druckmessung vorteilhaft oder erforderlich sein kann.

Bei vorbekannten Steuergeräten für Ballonkatheter wird der Druck unmittelbar in dem Druckmedium gemessen. Vorteilhaft ist es demgegenüber, wenn die Druckmeßeinheit einen Druckaufnehmer aufweist, der mit dem Zylinder und/oder dem Kolben in Wirkverbindung steht. Die Wirkverbindung kann derart ausgestaltet sein, daß der Druckaufnehmer mit dem Druckmedium des Ballonkatheters nicht in Berührung kommt, so daß der Druckaufnehmer oder Teile davon von diesem Druckmedium nicht kontaminiert werden können. In diesem Fall steht der Druckaufnehmer mit dem Zylinder und/oder dem Kolben in einer indirekten Wirkverbindung.

Vorteilhaft ist es, wenn der Druckaufnehmer der Druckmeßeinheit an dem Zylinderboden anliegt. Stattdessen oder zusätzlich kann es vorteilhaft sein, wenn der Druckaufnehmer an dem Kolben, insbesondere Kolbenende und/oder einer Antriebsstange für den Kolben anliegt. In diesen Fällen ist es möglich, daß der Druckaufnehmer an dem Zylinderboden bzw. dem Kolben bzw. dem Kolbenende bzw. der Antriebsstange unmittelbar anliegt. Es ist allerdings auch möglich, daß der Druckaufnehmer über eines oder mehrere Zwischenstücke an dem Zylinderboden bzw. dem Kolben bzw. dem Kolbenende bzw. der Antriebsstange anliegt.

Eine weitere vorteilhafte Weiterbildung ist dadurch gekennzeichnet, daß der Druckaufnehmer der Druckmeßeinheit als Membran ausgebildet ist. Ferner kann der Druckaufnehmer der Druckmeßeinheit als Zahnstange ausgebildet sein.

Nach einer weiteren vorteilhaften Weiterbildung ist die Kolben-Zylinder-Einheit in das Gehäuse einlegbar oder einsteckbar. Das Gehäuse kann eine Öffnung aufweisen, durch die die Kolben-Zylinder-Einheit in das Gehäuse einlegbar oder einsteckbar ist.

Vorteilhaft ist es, wenn in dem Gehäuse eine Antriebsstange längsverschieblich gelagert ist.

Stattdessen oder zusätzlich kann in dem Gehäuse eine Antriebsstange, die ein Gewinde aufweist, drehbar gelagert sein. Vorzugsweise handelt es sich dabei um die längsverschiebliche Antriebsstange. Eine weitere vorteilhafte Weiterbildung ist dadurch gekennzeichnet, daß in dem Gehäuse eine Gewindemutter vorgesehen ist, die in das Gewinde der Antriebsstange einrastbar und/oder aus dem Gewinde der Antriebsstange ausrastbar ist. Das Einrasten und/oder Ausrasten erfolgt vorzugsweise gegen eine Federkraft. Die Verwendung einer Gewindemutter ist insbesondere vorteilhaft, wenn die Antriebsstange auch längsverschieblich gelagert ist. In diesem Fall kann der schnelle Vorschub durch eine Längsverschiebung der Antriebsstange bei ausgerasteter Gewindemutter erfolgen, während die Feineinstellung durch eine Drehung der Antriebsstange bei eingerasteter Gewindemutter bewirkt werden kann.

Der Zylinderboden kann eine Abschlußplatte aufweisen, die in eine Aufnahme des Gehäuses einschiebbar ist. Die Aufnahme kann an einem Teil, das mit dem Gehäuse verbunden ist, beispielsweise einer Druckmeßeinheit, vorgesehen sein.

Vorzugsweise weist der Kolben eine Abschlußplatte auf, die in eine Aufnahme einer Antriebsstange einschiebbar ist. Die Aufnahme kann an einem Teil, das mit der Antriebsstange verbunden ist, beispielsweise einer Druckmeßeinheit, vorgesehen sein.

Eine weitere vorteilhafte Weiterbildung ist dadurch gekennzeichnet, daß der Zylinder Vorsprünge aufweist, mit denen er in einer Öffnung des Gehäuses lösbar verrastbar ist. Vorteilhaft ist es, wenn die Verrastung durch einen Bajonettverschluß erfolgt.

Nach einer weiteren vorteilhaften Weiterbildung ist an dem Gehäuse ein Schwenkhebel schwenkbar gelagert, durch den der Kolben und/oder eine Antriebsstange verschiebbar ist. Der Schwenkhebel ist vorzugsweise im Bereich eines an dem Gehäuse ausgebildeten Pistolengriffs angeordnet.

Vorteilhaft ist es, wenn an dem Schwenkhebel ein Mitnehmer vorgesehen ist. Der Mitnehmer kann ein Gewinde aufweisen, das mit einem Gewinde einer Antriebsstange in Eingriff bringbar ist.

Die Kolben-Zylinder-Einheit kann von Hand betätigt werden, insbesondere durch die beschriebenen Betätigungselemente, nämlich eine Antriebsstange oder einen Schwenkhebel. Es ist allerdings auch möglich, die Kolben-Zylinder-Einheit durch Motorkraft zu betreiben. Dementsprechend umfaßt das Steuergerät nach einer weiteren vorteilhaften Weiterbildung einen Motor zum Antrieb der Kolben-Zylinder-Einheit.

Vorteilhaft ist es, wenn das Steuergerät eine Anzeigeeinheit und/oder eine Bedieneinheit umfaßt. Die Anzeigeeinheit und/oder die Bedieneinheit können mit dem Steuergerät in einem einzigen Gehäuse integriert sein. Es ist allerdings auch möglich, die Anzeigeeinheit und/oder die Bedieneinheit in gesonderten Gehäusen unterzubringen.

Die Erfindung betrifft ferner eine Kolben-Zylinder-Einheit für ein Steuergerät für einen Ballonkatheter mit einem Zylinder, in dem ein Kolben verschieblich gelagert ist. Nach einer ersten Lösung weist der Zylinderboden eine Abschlußplatte auf, die in eine Aufnahme des Gehäuses des Steuergeräts oder eines damit verbundenen Teils, insbesondere des Gehäuses einer Druckmeßeinheit, einschiebbar ist. Nach einer weiteren Lösung weist der Kolben eine Abschlußplatte auf, die in eine Aufnahme einer Antriebsstange oder eines damit verbundenen Teils, insbesondere des Gehäuses einer Druckmeßeinheit, einschiebbar ist.

Vorteilhaft ist es, wenn der Zylinder Vorsprünge aufweist, mit denen er in einer Öffnung des Gehäuses lösbar verrastbar ist, wobei die Verrastung vorzugsweise durch einen Bajonettverschluß erfolgt.

Eine weitere vorteilhafte Weiterbildung ist dadurch gekennzeichnet, daß der Zylinder im Bereich des Zylinderbodens einen Anschlußstutzen aufweist.

Ausführungsbeispiele der Erfindung werden nachstehend anhand der beigefügten Zeichnung im einzelnen erläutert. In der Zeichnung zeigt
- Fig. 1: ein Steuergerät für einen Ballonkatheter in einer schematischen Seitenansicht,
- Fig. 2: eine Abwandlung des Steuergeräts gemäß Fig. 1,
- Fig. 3: einen Stellring für das Steuergerät gemäß Fig. 1 und 2 in einer Ansicht von vorne,
- Fig. 4: ein abgewandeltes Steuergerät für einen Ballonkatheter in einer Seitenansicht,
- Fig. 5: eine Abwandlung des Steuergeräts gemäß Fig. 4 vor der Betätigung,
- Fig. 6: das Steuergerät gemäß Fig. 5 nach der Betätigung,
- Fig. 7: eine Kolben-Zylinder-Einheit und einen Teil eines Steuergeräts vor der Verbindung in einer perspektivischen Ansicht,
- Fig. 8: die Kolben-Zylinder-Einheit gemäß Fig. 7 nach dem Einsetzen in das Steuergerät,
- Fig. 9: eine abgewandelte Kolben-Zylinder-Einheit in einer perspektivischen Darstellung von schräg vorne,
- Fig. 10: die Kolben-Zylinder-Einheit gemäß Fig. 9 in einer perspektivischen Ansicht von hinten,
- Fig. 11: ein Steuergerät mit einem Motor zum Antrieb der Kolben-Zylinder-Einheit und
- Fig. 12: eine Abwandlung des Steuergeräts gemäß Fig. 11.

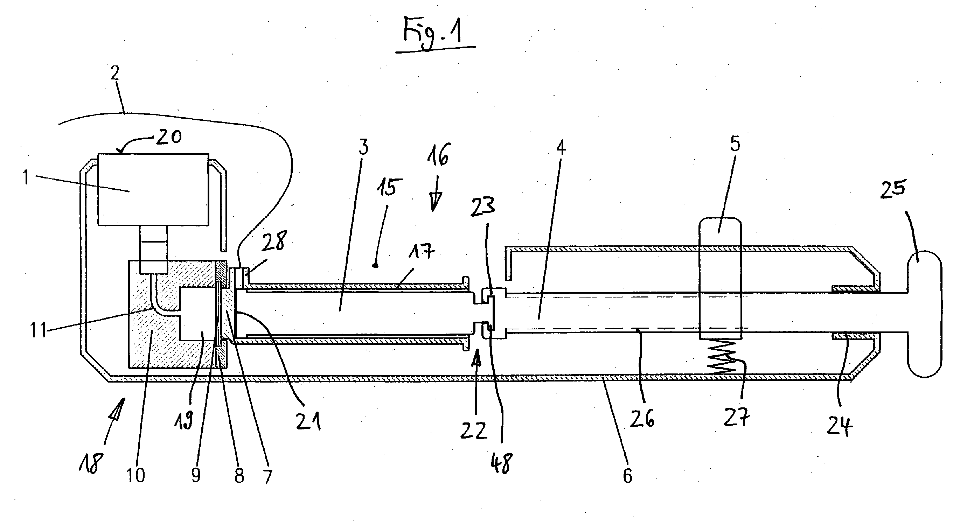

Das in Fig. 1 gezeigte Steuergerät für einen Ballonkatheter umfaßt ein Gehäuse 6 und eine Kolben-Zylinder-Einheit 16, die ihrerseits einen Kolben 3 und einen Zylinder 17 umfaßt. In dem Gehäuse 6 ist eine Öffnung 15 vorgesehen, durch die die Kolben-Zylinder-Einheit 16 in das Gehäuse 6 eingesetzt und aus dem Gehäuse 6 herausgenommen werden kann. Die Kolben-Zylinder-Einheit 16 ist mit dem Gehäuse 6 lösbar verbindbar.

In dem Gehäuse 6 ist eine Druckmeßeinheit 18 vorgesehen, die als Druckmeßdose ausgestaltet ist. Die Druckmeßeinheit 18 umfaßt ein Gehäuse 10 mit einer Vertiefung 19, deren Öffnung von einer Membran 9 verschlossen wird. Die Membran 9 wird von einem Deckel 8, der mit dem Gehäuse 10 verschraubt oder an dem Gehäuse 10 anderweitig befestigt sein kann, gehalten. An der Membran 9 liegt der Zylinderboden 7, also der Boden des Zylinders 17, an. Er kann in der aus Fig. 1 ersichtlichen Weise verdickt sein.

Von der Vertiefung 19 führt eine Druckleitung 11 durch das Gehäuse 10 zum Manometer 1, das an das Gehäuse 10 angeschraubt ist und dessen Anzeigefläche 20 im wesentlichen bündig mit einer Außenfläche des Gehäuses 6 abschließt. Die Vertiefung 19 und die Druckleitung 11 sind mit einer hydraulischen Flüssigkeit gefüllt, durch die der auf die Membran 9 ausgeübte Druck zum Manometer 1 übertragen wird.

Der in dem Zylinder 17 längsbewegliche Kolben 3 weist an seinem der Stirnfläche 21 abgewandten Ende ein T-förmiges Anschlußstück 22 auf, das aus dem Zylinder 17 herausragt und dessen Abschlußplatte 48 mit einer entsprechend geformten Aufnahme 23 einer Antriebsstange 4 lösbar verbindbar ist.

Die Antriebsstange 4 fluchtet mit dem Kolben 3. Sie ist in einem Lager 24 des Gehäuses 6 längsverschieblich und drehbar gelagert. An ihrem der Aufnahme 23 abgewandten, aus dem Gehäuse 6 herausragenden Ende ist die Antriebsstange 4 mit einem Handgriff 25 versehen. Sie weist ferner ein Gewinde 26 auf.

In dem Gehäuse 6 ist eine Gewindemutter 5 vorgesehen, die in das Gewinde 26 der Antriebsstange 4 einrastbar und aus diesem Gewinde 26 ausrastbar ist. Zu diesem Zweck ist die Gewindemutter 5 durch eine Druckfeder 27 vorbelastet, die sich zum einen an dem Gehäuse 6 und zum anderen an der Gewindemutter 5 abstützt. In dem in Fig. 1 gezeigten Zustand ist die Gewindemutter 5 in das Gewinde 26 der Antriebsstange 4 eingerastet. Ein Vorschub der Aufnahme 23 der Antriebsstange 4 und damit des Kolbens 3 kann durch eine Drehung am Handgriff 25 der Antriebsstange 4 erzeugt werden. Wenn ein schnellerer Vorschub des Kolbens 3 erreicht werden soll, kann die Gewindemutter 5 aus dem Gewinde 26 ausgerastet werden. Zu diesem Zweck weist die Gewindemutter 25 ein aus dem Gehäuse 6 herausragendes Ende auf, das der Druckfeder 27 gegenüberliegt. Durch einen Druck auf dieses Ende entgegen der Kraft der Druckfeder 27 wird die Gewindemutter 5 aus dem Gewinde 26 ausgerastet. Jetzt kann ein Vorschub des Kolbens 3 durch einen Druck auf den Handgriff 25 in Richtung zur Kolben-Zylinder-Einheit 16 hin erzeugt werden. Die Gewindemutter 5 kann eine Einrichtung aufweisen, durch die sie in der ausgerasteten Stellung gehalten wird (in der Zeichnung nicht dargestellt).

Im Betrieb wird die Kolben-Zylinder-Einheit 16 durch die Öffnung 15 in das Gehäuse 6 eingesetzt. In das Gehäuse 6 kann eine Kolben-Zylinder-Einheit 16 eingesetzt werden, die nicht mit einer zum Betrieb eines Ballonkatheters geeigneten Flüssigkeit gefüllt ist. In diesem Fall kann der Kolben 3 die in Fig. 1 dargestellte Stellung einnehmen, in der seine Stirnfläche 21 an dem Zylinderboden 7 anliegt. Die Abschlußplatte 48 des T-förmigen Anschlußstücks 22 des Kolbens 3 wird mit der Aufnahme 23 der Antriebsstange 4 verbunden. Ferner wird eine Quelle für Ballonkatheterflüssigkeit mit dem Anschlußstutzen 28 des Zylinders 17 verbunden (in der Zeichnung nicht dargestellt). Der Anschlußstutzen 28 befindet sich im Bereich des Zylinderbodens 7, und seine Durchgangsöffnung fluchtet teilweise mit dem Zylinderboden 7. Der Kolben 3 wird durch einen Zug am Handgriff 25 aufgezogen, so daß sich der Zylinder 17 mit Ballonkatheterflüssigkeit füllt. Danach wird der Anschlußstutzen 28 mit der Druckleitung 2 zum Ballonkatheter verbunden. Der Betrieb des Ballonkatheters kann beginnen. Während dieses Betriebs wird der Druck, der an der Anzeigefläche 20 des Manometers 1 angezeigt wird, überwacht. Der Betriebsdruck der Ballonkatheterflüssigkeit in der Kolben-Zylinder-Einheit 16 wirkt durch den Zylinderboden 7 hindurch auf die Membran 9 und durch diese hindurch auf die Hydraulikflüssigkeit in der Vertiefung 19, von der dieser Druck durch die Druckleitung 11 zum Manometer 1 geleitet und auf der Anzeigefläche 20 angezeigt wird. Der gemessene Druck kann auch weitergeleitet werden. Er kann insbesondere in ein elektrisches Signal umgewandelt und einer Auswerte- und/oder Anzeigeeinrichtung zugeführt werden, insbesondere einem Computer, der auch die Steuerung/oder Regelung des Betriebs des Ballonkatheters übernehmen kann. Dieser Betrieb kann nach Programmen, die im Computer abgespeichert sind, durchgeführt werden.

Es ist allerdings auch möglich, in das Gehäuse 6 eine Kolben-Zylinder-Einheit 16 einzusetzen, die bereits mit Ballonkatheterflüssigkeit gefüllt ist. Zu diesem Zweck kann die Öffnung 16 größer sein (in der Zeichnung nicht dargestellt).

Nach der Beendigung des Betriebs des Ballonkatheters kann die Druckleitung 2 vom Anschlußstutzen 28 gelöst werden. Die Kolben-Zylinder-Einheit 16 kann aus dem Gehäuse 6 herausgenommen und durch eine neue Kolben-Zylinder-Einheit 16 ersetzt werden.

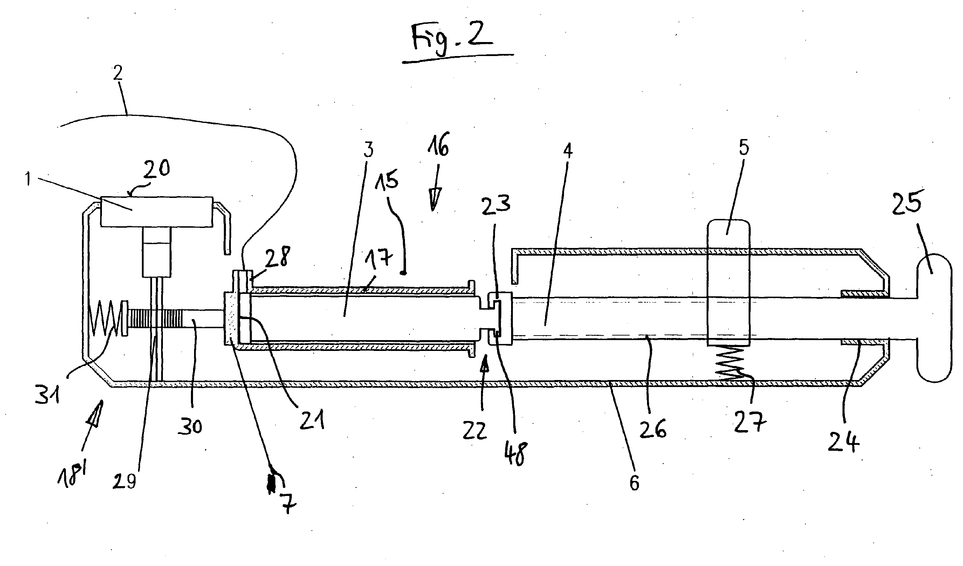

In Fig. 2 ist eine Abwandlung des Systems gemäß Fig. 1 gezeigt, in der übereinstimmende Bauteile mit denselben Bezugszeichen versehen sind. Im Unterschied zur Ausführungsform nach Fig. 1 ist bei der Abwandlung nach Fig. 2 keine hydraulische, sondern eine mechanische Druckmeßeinheit 18' vorgesehen, die eine Zahnstange 30 umfaßt, deren Ende an dem Zylinderboden 7 anliegt. Das andere Ende der Zahnstange 30 ist über eine Druckfeder 31 am Gehäuse 6 abgestützt. Die Zähne der Zahnstange 30 greifen in entsprechende Zähne einer Zahnwelle 29 ein, die rechtwinklig zur Zahnstange 30 verläuft und die mit einem Getriebe eines Manometers 1 verbunden ist, das an seiner Sichtfläche 20 den gemessenen Druck anzeigt. Bei der Abwandlung nach Fig. 2 umfaßt die Druckmeßeinheit 18' einen Druckaufnehmer, der an dem Zylinderboden 7 anliegt, nämlich die Zahnstange 30.

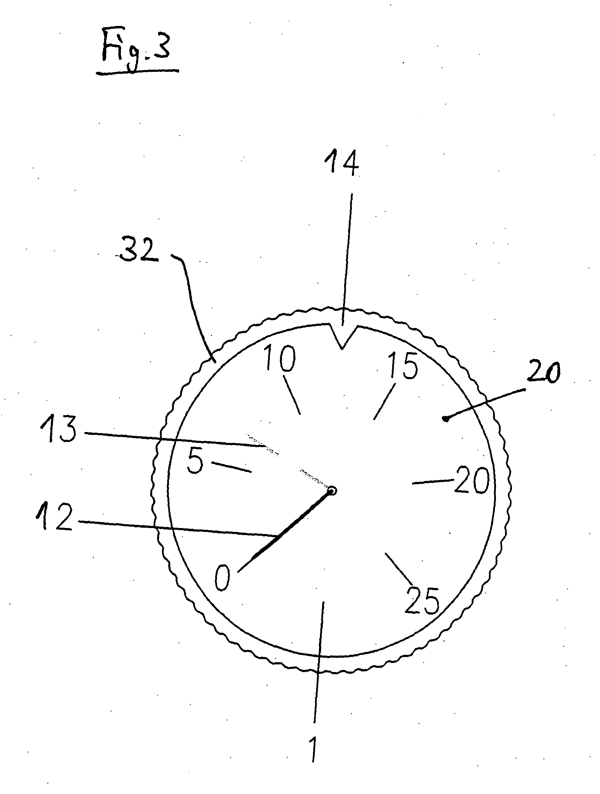

Fig. 3 zeigt die Sichtfläche 20 des Manometers 1, die von einem drehbaren Stellring 32 umgeben ist. Der Stellring 32 umfaßt eine nach innen weisende Markierung 14, die auf einen vorbestimmten Druck, beispielsweise einen gewünschten Druck oder einen höchstzulässigen Druck, eingestellt werden kann, im gewählten Beispiel auf einen Druck von 12,5 bar. Der Zeiger 12 zeigt zunächst einen Druck von 0 bar an. In der Stellung 13 wird ein Druck von 6,5 bar angezeigt. Die Markierung 14 kann mit einer Wameinrichtung zur Auslösung eines akustischen und/oder visuellen und/oder sonstigen Alarms bei Erreichen des durch diese Markierung eingestellten Drucks versehen sein.

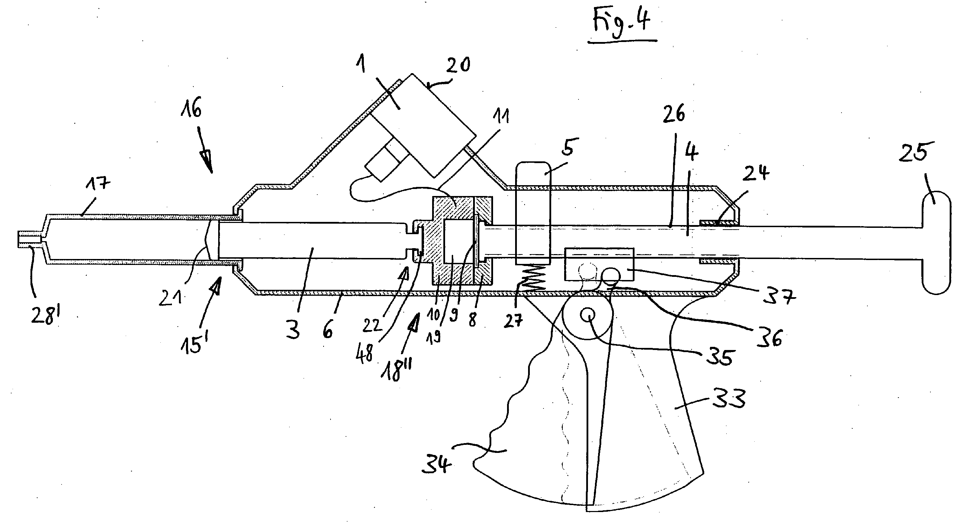

In Fig. 4 ist eine weitere Abwandlung eines Steuergeräts für einen Ballonkatheter gezeigt, bei der wiederum Bestandteile, die mit denjenigen der Ausführungsformen nach Fig. 1 und 2 übereinstimmen, mit denselben Bezugszeichen versehen sind. Bei der Ausführungsform nach Fig. 4 ist die Druckmeßeinheit 18" mit dem Kolben 3 verbunden. Sie liegt zwischen dem Kolben 3 und der Antriebsstange 4. Die Druckmeßeinheit 18" stimmt ansonsten mit derjenigen nach Fig. 1 überein. Sie umfaßt eine Membran 9, die durch einen Deckel 8 an dem Gehäuse 10 festgeklemmt ist. In der Vertiefung 19 befindet sich Hydraulikflüssigkeit, die durch die Druckleitung 11 zum Manometer 1 geleitet wird, welches den gemessenen Druck auf der Anzeigefläche 20, die im wesentlichen mit einer Außenfläche des Gehäuses 6 bündig abschließt, anzeigt.

Bei der Ausführungsform nach Fig. 4 liegt die dem Kolben 3 zugewandte Endfläche der Antriebsstange 4 an der Membran 9 der Druckmeßeinheit 18" an. Der Betriebsdruck der Ballonkatheterflüssigkeit, die sich in dem Zylinder 17 befindet, wird auf diese Weise auf die Membran 9 und die in der Vertiefung 19 befindliche Hydraulikflüssigkeit übertragen.

Das dem Kolben 3 zugewandte Ende der Antriebsstange 4 ist T-förmig ausgestaltet. Es liegt in einer entsprechenden Vertiefung innerhalb des Deckels 8, durch die es dort festgehalten wird. Die Verbindung zwischen dem Kolben 3 und der Antriebsstange 4 wird durch die Druckmeßeinheit 18" hergestellt.

Der Zylinder 17 ist an seinem vorderen Ende mit einem zentralen Anschlußstutzen 28' versehen, an den die zum Ballonkatheter führende Druckleitung angeschlossen werden kann (in der Zeichnung nicht dargestellt).

Das Gehäuse 6 ist im hinteren unteren Bereich mit einem Pistolengriff 33 versehen, an dem ein Schwenkhebel 34 schwenkbar gelagert ist. Die Schwenkachse 35 befindet sich im oberen vorderen Bereich des Pistolengriffs 33, etwas unterhalb der Unterseite des Gehäuses 6. Der Schwenkhebel 34 weist auf der der Schwenkachse 35 gegenüberliegenden Seite einen Betätigungshebel 36 auf, dessen zylinderförmiger oder kugelförmiger Endbereich in eine entsprechende Vertiefung in einem Mitnehmer 37 eingreift. In die Oberseite des Mitnehmers 37 ist ein Innengewinde eingearbeitet, das dem Außengewinde der Antriebsstange 4 entspricht, das sich jedoch über einen Umfangsbereich von höchstens 180° erstreckt. Der Schwenkhebel 34 ist durch eine Feder (in der Zeichnung nicht dargestellt) derart vorbelastet, daß er die in Fig. 4 durchgezogen gezeichnete Stellung einnimmt.

Wenn der Schwenkhebel 34 entgegen dieser Federbelastung gedrückt wird, wird er um die Schwenkachse 35 in einer Richtung entgegen dem Uhrzeigersinn gedreht. Das Ende des Betätigungshebels 36 bewegt sich geringfügig nach oben und in Richtung zum Kolben 3 hin. Hierdurch gelangt das Teil-Innengewinde des Mitnehmers 37 mit dem Außengewinde der Antriebsstange 4 in Eingriff, und die Antriebsstange 4 drückt den Kolben 3 in den Zylinder 17. Der dabei entstehende Druck wird an der Membran 9 abgegriffen und vom Manometer 1 angezeigt. Um die Bewegung der Antriebsstange 4 zu ermöglichen, muß die Gewindemutter 5 entgegen der Kraft der Feder 27 gedrückt sein, um das Innengewinde der Gewindemutter 5 vom Außengewinde 26 der Antriebsstange 4 auszurasten. Anderenfalls ist die Antriebsstange 4 gesperrt; sie kann dann durch Drücken des Schwenkhebels 34 nicht in Längsrichtung bewegt werden.

Der Eingriff des Mitnehmers 37 in die Antriebsstange 4 kann derart ausgestaltet sein, daß eine repetitive Betätigung möglichst ist. Dann wird durch jede Betätigung des Schwenkhebels 34 entgegen dem Uhrzeigersinn ein Vorschub des Kolbens 3 erzeugt. Wenn der Schwenkhebel 34 losgelassen wird, so daß er sich in Richtung des Uhrzeigersinns bewegt, erfolgt in diesem Fall keine Rückbewegung der Antriebsstange 4.

Bei der Ausführungsform nach Fig. 4 wird die Kolben-Zylinder-Einheit 16 durch eine Öffnung 15' in das Gehäuse 16 eingesetzt, die im wesentlichen der Außenumfangsfläche bzw. dem Außendurchmesser des Zylinders 17 entspricht.

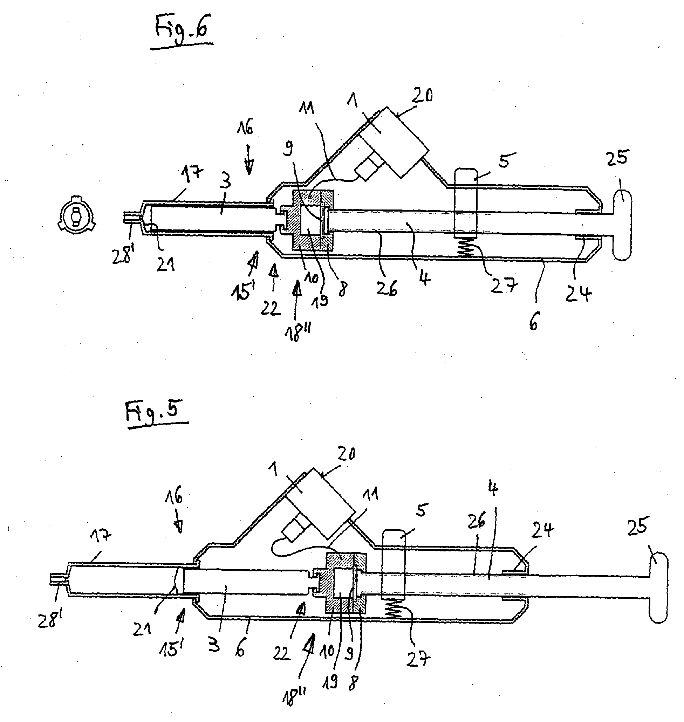

Fig. 5 zeigt das Steuergerät gemäß Fig. 4, allerdings ohne Pistolengriff 33, in der Stellung vor Beginn des Betriebes des Ballonkatheters, in der sich der Kolben 3 in der zurückgezogenen Stellung befindet. Während des Betriebes des Ballonkatheters wird der Kolben 3 in den Zylinder 17 hinein bewegt, in der Darstellung der Fig. 5 also in Richtung nach links, bis die in Fig. 6 gezeigte Stellung eingenommen wird. Während dieses Betriebes wird der Druck im Ballonkatheterfluid, also innerhalb des Zylinders 17, laufend gemessen, wobei der Druckaufnehmer von der Membran 9 gebildet wird.

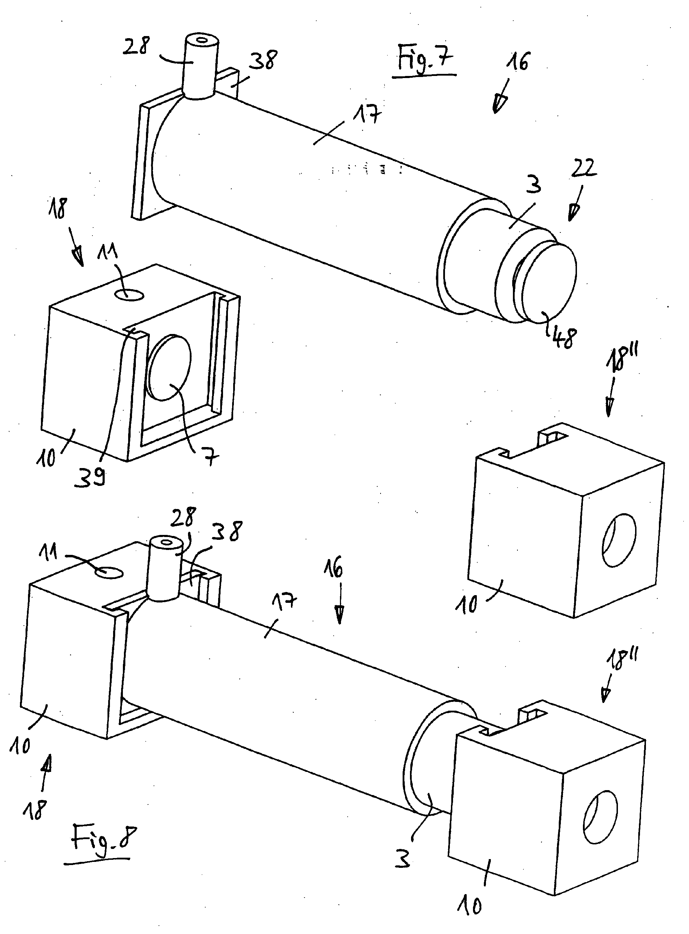

Fig. 7 zeigt eine Kolben-Zylinder-Einheit 16, die im wesentlichen derjenigen nach Fig. 1 und 2 entspricht und bei der übereinstimmende Teile mit denselben Bezugszeichen versehen sind. Der Zylinderboden 7 ist mit einer quadratischen Abschlußplatte 38 versehen, die in eine entsprechend gestaltete Aufnahme 39 des Gehäuses 10 der Druckmeßeinheit 18 einschiebbar ist. Die Aufnahme 39 umfaßt 2 parallele Nuten, in denen die Ränder der quadratischen Anschlußplatte 38 aufgenommen werden.

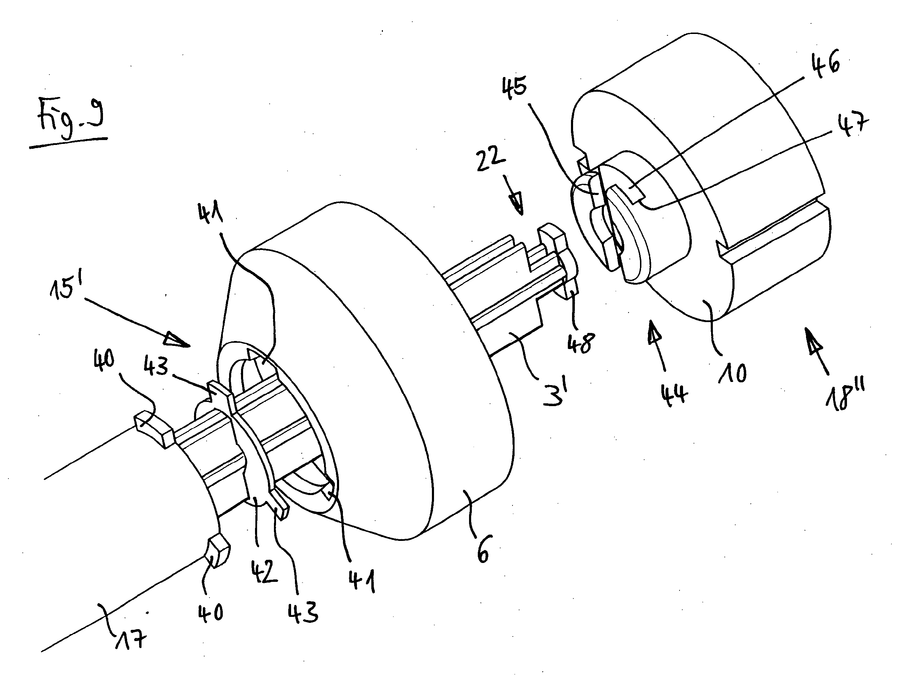

Fig. 8 zeigt die Kolben-Zylinder-Einheit 16 im eingeschobenen Zustand, in dem die Rückseite der quadratischen Anschlußplatte 38 an der Vorderseite der Membran 7 anliegt.

In Fig. 7 ist ferner eine Druckmeßeinheit 18" gezeigt, die im wesentlichen derjenigen der Figuren 4 bis 6 entspricht. Aus Gründen der vereinfachten zeichnerischen Darstellung sind in den Fig. 7 und 8 sowohl die Druckmeßeinheit 18 als auch die Druckmeßeinheit 18" dargestellt. Bei einem realisierten Gerät ist es vorteilhaft, nur eine dieser beiden Druckmeßeinheiten 18 oder 18" anzuwenden. Das Gehäuse 10 der Druckmeßeinheit 18" umfaßt eine Aufnahme, die im wesentlichen derjenigen der Druckmeßeinheit 18 entspricht und in die die Abschlußplatte 48 des T-förmigen Anschlußstücks 22 des Kolbens 3 eingeschoben werden kann. Die montierte Stellung ist in Fig. 8 gezeigt.

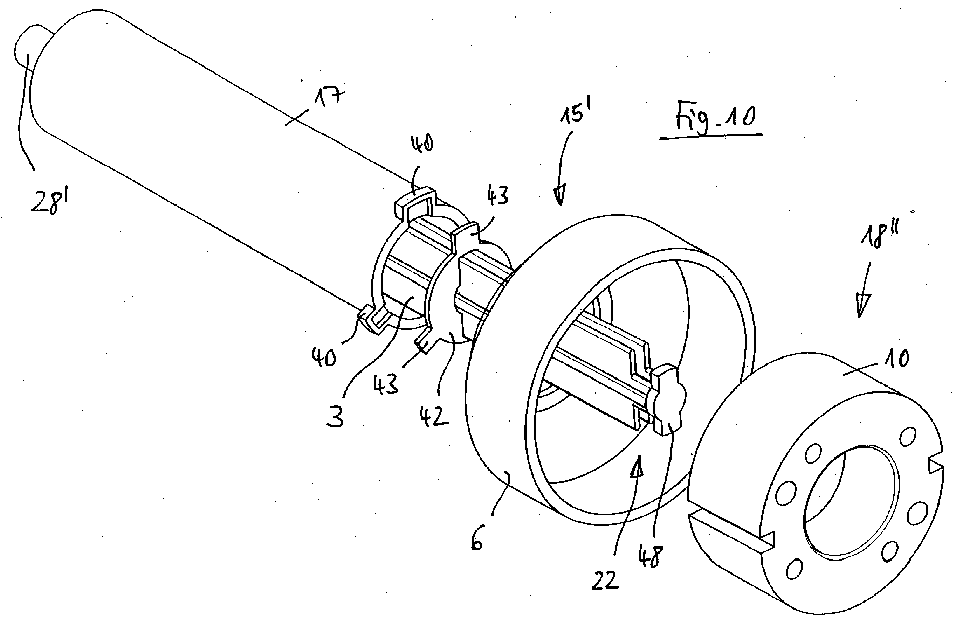

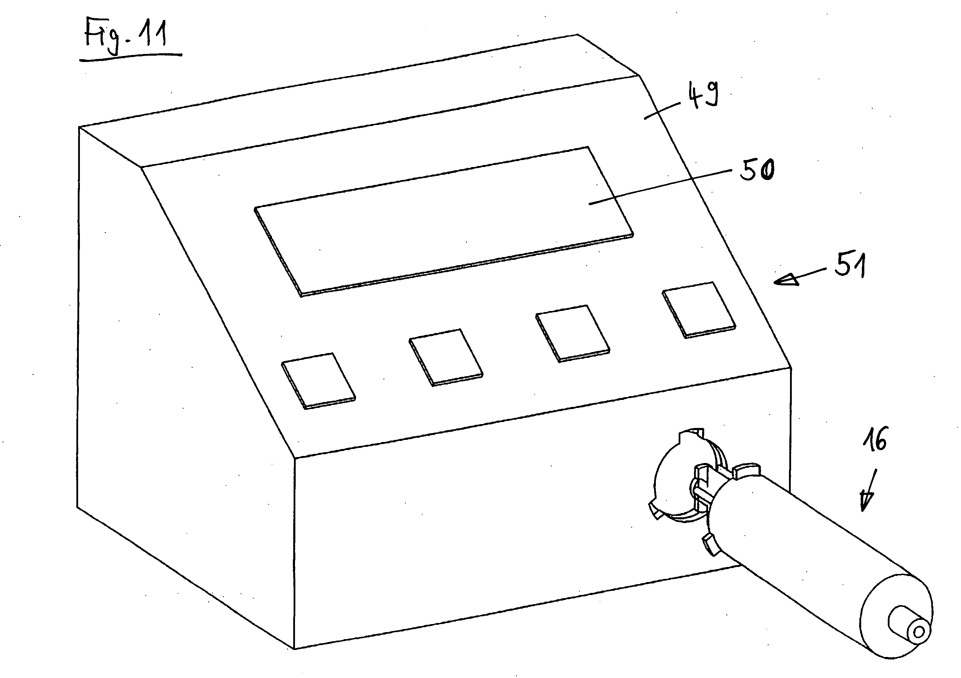

Fig. 9 und 10 zeigen einen Teil der Ausführungsform nach Fig. 4 bis 6, wobei übereinstimmende Teile mit denselben Bezugszeichen versehen sind. Der Zylinder 17 ist an seinem hinteren Ende mit Vorsprüngen 40 versehen. Am Rand der Öffnung 15' des Gehäuses 6 sind entsprechende Aussparungen 41 vorhanden. Die Anordnung und/oder Ausgestaltung der Vorsprünge 40 und Aussparungen 41 ist derart getroffen, daß der Zylinder 17 nur in einer bestimmten Stellung in die Öffnung 15' im Gehäuse 6 eingeschoben werden kann. Hinter den Aussparungen 41 befinden sich Umfangsnuten, die sich über einen bestimmten Winkelbereich erstrecken und deren Enden Anschläge bilden, so daß der Zylinder 17 in die Öffnung 15' eingeschoben und durch eine anschließende Drehung nach Art eines Bajonettverschlusses arretiert werden kann.

Der Kolben 3' besitzt einen langgestreckten Querschnitt. Auf dem Kolben 3' ist eine Verdrehsicherung 42 längsverschieblich gelagert, deren innere Aussparung der Außenkontur des Kolbens 3' entspricht und deren Außenkontur derjenigen des Zylinders 17 im Bereich der Vorsprünge 40 entspricht. Nach dem Einschieben des Zylinders 17 in die Öffnung 15' kommen die den Vorsprüngen 40 entsprechenden Vorsprünge 43 der Verdrehsicherung 42 in entsprechenden Aussparungen innerhalb des Gehäuses 6 zu liegen, so daß der Kolben 3' in dieser Stellung gegen Verdrehen gegenüber dem Gehäuse 6 gesichert ist.

Das T-förmige Anschlußstück 22 des Kolbens 3' kann in eine Aufnahme 44 im Gehäuse 10 der Druckmeßeinheit 18" eingeschoben und dort arretiert werden. Hierzu umfaßt die Aufnahme 44 einen Längsschlitz 45, durch den hindurch die Abschlußplatte 48 des T-förmigen Anschlußstücks 22 geschoben werden kann. Nach dem Durchtritt durch den Längsschlitz 45 kann sie durch eine Drehung in der dahinter befindlichen Umfangsnut 46 bis zum Anschlag 47 nach Art eines Bajonettverschlusses arretiert werden.

Durch die Erfindung besteht die Möglichkeit, ein resterilisierbares Steuergerät für eine auswechselbare Zylinder-Kolben-Einheit zur Inflation und/oder Deflation von Ballonkathetern zu realisieren. Die Kolben-Zylinder-Einheit kann nach Gebrauch entfernt werden, und das Steuergerät, das mit dem Ballonkatheterfluid nicht in Kontakt gekommen ist, kann sterilisiert werden. Mit dem Ballonkatheterfluid kommen nur der Zylinder, der Kolben und die Druckleitung zum Ballonkatheter in Kontakt. Die Membran 9 ist aus elastischem Material gefertigt, insbesondere aus Kunststoff, beispielsweise PTFE, oder aus Metall.

Die Messung des Drucks im Ballonkatheterfluid erfolgt nicht durch eine Messung im Zylinder, sondern durch eine indirekte Messung durch eine Membran (hydraulisch) oder mechanisch oder elektronisch. Die Druckmessung kann durch einen Druckabgriff am Zylinderboden oder am Kolben oder an einer Antriebsstange für den Kolben erfolgen. Es ist möglich, den Druckabgriff im oder am Gewinde der Antriebsstange zu realisieren. Es wird keine interne Druckmessung (im Inneren des Zylinders unmittelbar am Ballonkatheterfluid), sondern eine externe Druckmessung durchgeführt.

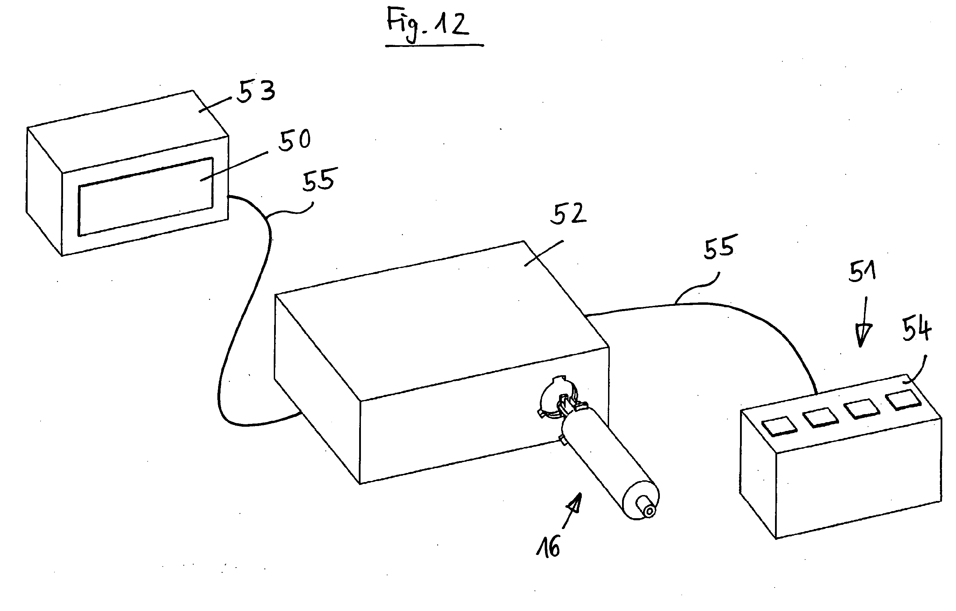

Fig. 11 zeigt ein Steuergerät mit einem Gehäuse 49, in dem ein Motor, nämlich ein Elektromotor, zum Antrieb der Kolben-Zylinder-Einheit 16 vorgesehen ist. Das Steuergerät umfaßt ferner eine Anzeigeeinheit 50 und eine Bedieneinheit 51, die mehrere Bedienungstasten umfaßt. Es sind insgesamt vier Bedienungstasten vorgesehen, und zwar für eine langsame und schnelle Vorwärtsbewegung des Kolbens und eine langsame und schnelle Rückwärtsbewegung des Kolbens. Es ist allerdings auch eine andere Tastenbelegung möglich. Ferner können auch mehr oder weniger als vier Bedienungstasten vorgesehen sein. Wie aus Fig. 11 ersichtlich wird der Zylinder der Kolben-Zylinder-Einheit mit dem Gehäuse 46 verrastet. Der Elektromotor wird an den Kolben angekuppelt. Er betätigt dann den Kolben. Die Anzeigeeinheit 50 und die Bedieneinheit 51 sind in das Gehäuse 49 des Steuergeräts integriert.

Bei der in Fig. 12 gezeigten Abwandlung umfaßt das Steuergerät ein erstes Gehäuse 52, in dem ein Motor zum Antrieb der Kolben-Zylinder-Einheit 16 vorgesehen ist, ein zweites Gehäuse 53, in dem die Anzeigeeinheit 50 vorgesehen ist, und ein drittes Gehäuse 54, in dem die Bedieneinheit 51 vorgesehen ist. Die Anzeigeeinheit 50 und die Bedieneinheit 51 bzw. deren Gehäuse 53, 54 sind durch Kabel 55 mit dem Gehäuse 52 des Steuergeräts verbunden.

## Patentansprüche

1. Steuergerät für einen Ballonkatheter,
**gekennzeichnet durch**
ein Gehäuse (6) und eine Kolben-Zylinder-Einheit (16), die mit dem Gehäuse (6) lösbar verbindbar ist.

2. Steuergerät nach Anspruch 1, **dadurch gekennzeichnet, daß** in dem Gehäuse (6) eine Druckmeßeinheit (18, 18', 18") vorgesehen ist.

3. Steuergerät nach Anspruch 2, **dadurch gekennzeichnet, daß** die Druckmeßeinheit (18, 18', 18") einen Druckaufnehmer (9, 30) aufweist, der mit dem Zylinder (17) und/oder dem Kolben (3) in Wirkverbindung steht.

4. Steuergerät nach Anspruch 2 oder 3, **dadurch gekennzeichnet, daß** der Druckaufnehmer der Druckmeßeinheit an dem Zylinderboden (7) und/oder an dem Kolben (3) und/oder an einer Antriebsstange (4) anliegt.

5. Steuergerät nach einem der Ansprüche 2 bis 4, **dadurch gekennzeichnet, daß** der Druckaufnehmer der Druckmeßeinheit als Membran (9) und/oder als Zahnstange (30) ausgebildet ist.

6. Steuergerät nach einem der vorhergehenden Ansprüche, **dadurch gekennzeichnet, daß** die Kolben-Zylinder-Einheit (16, 16') in das Gehäuse (6) einlegbar oder einsteckbar ist.

7. Steuergerät nach einem der vorhergehenden Ansprüche, **dadurch gekennzeichnet, daß** in dem Gehäuse (6) eine Antriebsstange (4) längsverschieblich gelagert (24) ist.

8. Steuergerät nach einem der vorhergehenden Ansprüche, **dadurch gekennzeichnet, daß** in dem Gehäuse (6) eine Antriebsstange (4), die ein Gewinde (26) aufweist, drehbar gelagert (24) ist, wobei in dem Gehäuse (6) vorzugsweise eine Gewindemutter (5) vorgesehen ist, die in das Gewinde (26) der Antriebsstange (4) einrastbar und/oder aus dem Gewinde (26) der Antriebsstange (4) ausrastbar ist.

9. Steuergerät nach einem der vorhergehenden Ansprüche, **dadurch gekennzeichnet, daß** der Zylinderboden (7) eine Abschlußplatte (38) aufweist, die in eine Aufnahme (39) des Gehäuses (6) des Steuergeräts oder des Gehäuses (10) der Druckmeßeinheit (18, 18') einschiebbar ist.

10. Steuergerät nach einem der vorhergehenden Ansprüche, **dadurch gekennzeichnet, daß** der Kolben (3) eine Abschlußplatte (48) aufweist, die in eine Aufnahme (23) einer Antriebsstange (4) oder des Gehäuses (10) der Druckmeßeinheit (18") einschiebbar ist.

11. Steuergerät nach einem der vorhergehenden Ansprüche, **dadurch gekennzeichnet, daß** der Zylinder (17) Vorsprünge (40) aufweist, mit denen er in eine Öffnung (15') des Gehäuses (6) lösbar verrastbar ist.

12. Steuergerät nach einem der vorhergehenden Ansprüche, **dadurch gekennzeichnet, daß** an dem Gehäuse (6) ein Schwenkhebel (34) schwenkbar gelagert ist, durch den der Kolben (3) und/oder eine Antriebsstange (4) verschiebbar ist, wobei an dem Schwenkhebel (34) vorzugsweise ein Mitnehmer (37) vorgesehen ist.

13. Steuergerät nach einem der vorhergehenden Ansprüche, **gekennzeichnet durch** einen Motor zum Antrieb der Kolben-Zylinder-Einheit (16).

14. Steuergerät nach einem der vorhergehenden Ansprüche, **gekennzeichnet durch** eine Anzeigeeinheit (50) und/oder eine Bedieneinheit (51).

15. Kolben-Zylinder-Einheit für ein Steuergerät für einen Ballonkatheter mit einem Zylinder (17), in dem ein Kolben (3) verschieblich gelagert ist,
**dadurch gekennzeichnet,**
**daß** der Zylinderboden (7) eine Abschlußplatte (38) aufweist, die in eine Aufnahme (39) des Gehäuses (6) des Steuergeräts oder eines damit verbundenen Teils, insbesondere des Gehäuses (10) einer Druckmeßeinheit (18, 18") einschiebbar ist.

16. Kolben-Zylinder-Einheit für ein Steuergerät für einen Ballonkatheter mit einem Zylinder (17), in dem ein Kolben (3) verschieblich gelagert ist,
**dadurch gekennzeichnet,**
**daß** der Kolben (3) eine Abschlußplatte (48) aufweist, die in eine Aufnahme (23) einer Antriebsstange (4) oder eines damit verbundenen Teils, insbesondere des Gehäuses (10) einer Druckmeßeinheit (18") einschiebbar ist.

17. Kolben-Zylinder-Einheit nach Anspruch 15 oder 16, **dadurch gekennzeichnet, daß** der Zylinder (17) Vorsprünge (40) aufweist, mit denen er in eine Öffnung (15') des Gehäuses (6) lösbar verrastbar ist.

18. Kolben-Zylinder-Einheit nach einem der Ansprüche 15 bis 17, **dadurch gekennzeichnet, daß** der Zylinder (17) im Bereich des Zylinderbodens (7) einen Anschlußstutzen (28) aufweist.
